(19) Europäisches Patentamt
European Patent Office
Office européen des brevets

(11) Publication number: **0 093 551 B1**

(12) **EUROPEAN PATENT SPECIFICATION**

(45) Date of publication of patent specification: **16.01.91**

(21) Application number: **83302290.8**

(22) Date of filing: **22.04.83**

(51) Int. Cl.$^5$: **A 61 K 45/06,** A 61 K 37/26, C 07 C 271/22, C 07 C 233/45, C 07 K 5/02 // (A61K37/26, 31:27, 31:195, 31:165)

(54) **Pharmaceutical composition.**

(30) Priority: **30.04.82 JP 73306/82**
**14.04.83 JP 65999/83**

(43) Date of publication of application:
**09.11.83 Bulletin 83/45**

(45) Publication of the grant of the patent:
**16.01.91 Bulletin 91/03**

(84) Designated Contracting States:
**DE FR GB**

(56) References cited:
**GB-A-2 095 994**
**CHEMICAL ABSTRACTS, vol. 95, no. 24, December 14, 1981, page 374, abstract no. 209609m, Columbus, Ohio, US; A. KAMADA et al.: "Study of enamine derivatives on phenylglycine as adjuvants for the rectal absorption of insulin"**
**CHEMICAL ABSTRACTS, vol. 100, no. 2, January 9, 1984, page 362, abstract no 12583p, Columbus, Ohio, US; T. YAGI et al.: "Insulin suppository: enhanced rectal absorption of insulin using an enamine derivative as a new promoter"**

(73) Proprietor: **AJINOMOTO CO., INC.**
**5-8, Kyobashi 1-chome, Chuo-ku**
**Tokyo 104 (JP)**

(72) Inventor: **Toyoshima, Shigeshi**
**No. 3-14-22, Funabashi**
**Setagaya-ku Tokyo (JP)**
Inventor: **Fukushima**
**No. 2-1-10-406, Toyogaoka**
**Tama-shi Tokyo (JP)**
Inventor: **Seto, Yoshiko**
**No. 1-2-3-406, Natsumidai**
**Funabashi-shi Chiba-ken (JP)**
Inventor: **Kumashiro, Izumi**
**No. 1071-17, Ichigao-cho Midori-ku**
**Yokohama-shi Kanagawa-ken (JP)**

(74) Representative: **Bond, Bentley George et al**
**Haseltine Lake & Co. 28 Southampton Buildings**
**Chancery Lane**
**London WC2A 1AT (GB)**

(56) References cited:
**CHEMICAL ABSTRACTS, vol. 101, no. 15, October 8, 1984, page 108 abstract no 123533n, Columbus, Ohio, US; K. FUKUSHIMA et al.: "Oral administration of insulin in combination with amino acid derivatives in animal experiments"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 093 551 B1**

Courier Press, Leamington Spa, England.

**Description**

This invention relates to novel phenylalanine derivatives, and to pharmaceutical compositions comprising at least one of the phenylalanine derivatives as an absorption promoter, and insulin. In accordance with the invention the absorption of insulin in oral or rectal administration is improved.

The phenylalanine derivatives according to the invention are compounds represented by the general formula (I):

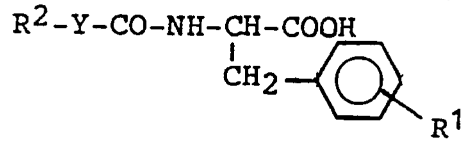

wherein $R^1$ is a hydrogen atom, a nitro group, a hydroxyl group or a hydroxyl group protected by an esterifying group, —Y— is a straight bond (i.e. the group $R^2$—Y—CO— is R—CO—), a $C_1$—$C_{10}$ alkylene group, a vinylene group, or a group having the formula —$CH_2$—O— or —O—$CH_2$— an $R^2$ is a phenyl or naphthyl group; or the group $R^2$—Y—CO— is an N-benzyloxycarbonylphenylalanyl group; or a non-toxic salt thereof.

The present invention also provides a pharmaceutical composition comprising (a) insulin and (b) a phenylalanine derivative of formula (I) or a non-toxic salt thereof.

When Y is a vinylidene group, this vinylidene group may contain substituents. When $R^2$ is a phenyl or naphthyl group, this phenyl or naphthyl group may contain substituents.

An example of a hydroxyl group protected by an esterifying group is a benzyloxycarbonyl group. Examples of the group $R^2$ include phenyl and naphthyl groups which may have, as a substituent, a halogen atom (such as a chlorine or fluorine atom), a nitro group, a lower alkyl group (such as a methyl or trifluormethyl group) or a lower alkyloxy group (such as a methoxy group).

The term "lower" herein usually means a group having up to 4 carbon atoms, but includes a group having up to about 10 carbon atoms such as a group having up to 8 carbon atoms or up to 6 carbon atoms.

Examples of compounds of formula (I) are:

S—1: N-[(1-naphthyl)acetyl]-L-phenylalanine
S—2: N-[(1-nitrophenoxy)acetyl]-4-nitro-DL-phenylalanine
S—3: N-cinnamoyl-L-phenylalanine
S—4: N,O-bis-(benzyloxycarbonyl)-L-tyrosine
S—5: N-phenoxyacetyl-L-phenylalanine
S—6: N-phenoxyacetyl-4-nitro-L-phenylalanine
S—7: N-benzyloxycarbonyl-L-phenylalanyl-L-phenylalanine
S—8: N-(4-phenylbutyroyl)-L-phenylalanine
S—9: N-phenoxyacetyl-D-phenylalanine
S—10: N-benzyloxycarbonyl-L-phenylalanyl-L-tyrosine
S—11: N-[(1-naphthoxy)acetyl]-L-phenylalanine
S—12: N-[(2-naphthoxy)acetyl]-L-phenylalanine
S—13: N-[(4-chlorophenoxy)acetyl]-L-phenylalanine
S—14: N-benzyol-L-phenylalanine
S—15: N-benzyloxycarbonyl-L-phenylalanine
S—16: N-benzyloxycarbonyl-L-tyrosine
S—17: N-(4-methoxybenzyloxycarbonyl-L-phenylalanine
S—18: N-benzyloxycarbonyl-D-phenylalanine
S—19: N-(4-fluorobenzylcarbonyl)-L-phenylalanine
S—20: N-benzyloxycarbonyl-D-phenylalanyl-L-phenylalanine
S—21: N-benzyloxycarbonyl-L-phenylalanyl-D-phenylalanine
S—24: N-(4-chlorocinnamoyl)-L-phenylalanine
S—25: N-(4-chlorocinnamoyl)-D-phenylalanine
S—26: N-(4-fluorocinnamoyl)-L-phenylalanine
S—27: N-(4-methylcinnamoyl)-L-phenylalanine
S—28: N-(4-trifluoromethylcinnamoyl)-L-phenylalanine
S—29: N-(3-methoxycinnamoyl)-L-phenylalanine
S—30: N-(3-trifluoromethylcinnamoyl)-L-phenylalanine
S—31: N-(α-fluorocinnamoyl)-L-phenylalanine
S—35: N-(4-trifluoromethylcinnamoyl)-D-phenylalanine
S—36: N-(3-trifluoromethylcinnamoyl)-D-phenylalanine

The above code numbers S—1 to S—21, S—24 to S—31, S—35 and S—36 are our own code numbers. These code numbers are used in the description hereinbelow.

The compounds having formula (I) are unique in that they can be used to promote the absorption of insulin. Phenylalanine *per se*, N-acetylphenylalanine and lower alkyl esters or amides of the acids of formula (I), are not useful as absorption promoters. The compounds of formula (I) have a symmetric carbon atoms and may be in D-form, in L-form or in DL-form depending upon the specific combination of

substituents. The phenylalanine derivatives of the present invention are novel, and can be prepared by conventional N-acylation techniques, for example by reaction of compound having the formula:

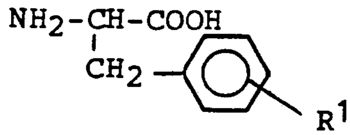

wherein $R^1$ is as defined above, with a compound having the formula:

$$R^2—Y—CO—Cl$$

wherein $R^2$ and Y are as defined above.

The phenylalanine derivatives of the present invention may be in a form of a salt, such as a metal salt (for example a sodium, potassium, lithium or calcium salt) or a salt with an organic base which is pharmaceutically acceptable. As the organic base, there can be adopted amines such as ammonia (ammonium salt), dicyclohexylamine and N-methyl-D-glucamine, and basic amino acids such as lysine and arginine.

The phenylalanine derivative of the present invention is administered orally or rectally, together with the insulin. Not only does the derivative promote the absorption of insulin but it also inhibits the degradation of insulin in the presence of trypsin and chymotrypsin.

Particularly, the oral or rectal administration of insulin for the treatment of diabetes mellitus has not been clinically established yet, and the development of an insulin-absorption promoter that permits extended and convenient use for humans is needed.

The absorption promoter of the present invention, i.e. the phenylalanine derivative, is usually employed on, the range of from 0.1—2000 mg, preferably 0.2 to 500 mg, to 250 of the insulin.

The absorption promoter may be administered in composition form with the insulin.

Regarding the form of the composition, the phenylalanine derivative can be used by formulating it into a preparation such as a tablet, a capsule, an elixir solution, a suspension, etc.

The phenylalanine derivative and the insulin can be administered to a human subject necessitating such treatment in a dosage range of from 0.1 to 1000 mg per subject, generally several times a day, that is, in a total daily dosage of 0.2 to 2000 mg. The dosage varies according to the seriousness of disease, the body weight of the subject, and other factors known by those skilled in the art.

The foregoing typical combinations of drug are formulated into pharmaceutical compositions as stated below. Usually 0.2 to 500 mg of the phenylalanine derivative and the insulin are blended into a unit dosage form generally acknowledged or required for pharmaceutical practice, together with a pharmaceutically acceptble vehicle, carrier, excipient, binder antiseptic, stabilizer, flavouring, and so forth. The amount of each active substance in these compositions or preparations is adjusted in such a way as to give an appropriate dosage of the prescribed range.

Specific materials which can be incorporated into tablets, capsules, and so forth are as follows: binders such as traganth, gum arabic, cornstarch, and gelatin; excipients such as microcrystalline cellulose; swelling agents such as cornstarch, pregelatinized starch and arginic acid; lubricants such as magnesium stearate; sweeteners such as sucrose, lactose and saccharin; and flavourings such as peppermint, oil from Gaultheria adenothrix Maxim, and cherry. Enteric coating is preferably carried out. For example, hydroxyphenylmethylcellulose (8%) aqueous solution as a pre-coating agent for forming an undercoat and hydroxypropylmethylcellulose phthalate (10%) and polyacetyne (3%) aqueous solution as a coating agent, may be used. When the unit dosage form of the preparation is a capsule, a liquid carrier such as a fatty oil can further be incorporated in the foregoing types of materials. Various other materials can be present as a coating material or in order to vary the physical form of the unit dosage forms according to other methods. For example, tablets can be coated with shellac and/or sugar. Syrups or elixirs can contain an active compound, sucrose as a sweetener, methylparaben or propylparaben as antiseptics, a colouring agent, or a flavouring agent such as cherry and orange flavouring.

Aceptic compositions can be formulated according to the usual practice for the preparation of pharmaceutical dosage forms, in which practice an active substance is dissolved or suspended in a vehicle such as water.

A buffer, antiseptic, or an antioxidant can further be incorporated as the occasion demands.

The following Examples illustrate the invention:

Example 1

Production of compound "S—6"·

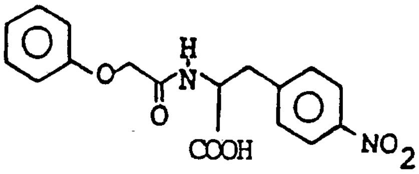

4-Nitro-L-phenylalanine (21 g) was dissolved in 10% naOH (10 ml), and an ethyl ether solution of phenoxyacetyl chloride (1.7 g) and an aqueous $Na_2CO_3$ solution prepared from $Na_2CO_3$ (2.7 g) and water (25 ml) were alternatively added stepwise thereto while stirring at room temperature over a period of 20 minutes. After that, the mixture was stirred at room temperature for 3 hours, and then acidified with dilute HCl to precipitate crystals. The crystals were filtered, washed with water, and re-crystallized from dioxan to obtain N-phenoxyacetyl-4-nitro-L-phenylalanine (2.4 g) as needles having a melting point of 147°C (2.4 g).

Elementary analysis:
Calc. C 59.30%, H 4.68%, N 8.14%
Found C 59.47%, H 4.51%, N 8.03%.

Example 2

Production of compound "S—8":

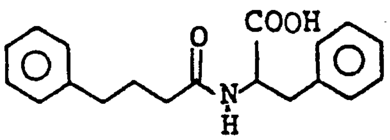

4-Phenylbutyric acid (25 g) was dissolved in chloroform (500 ml), and N-hydroxysuccinimide (17.3 g) was added thereto. N,N'-dicylohexylcarbodiimide (31 g) was added in portions to the mixture with ice-cooling while stirring. The mixture was stirred for 1 hour under cooling and then for 7 hours at room temperature. After the addition of glacial acetic acid (10 ml), the mixture was stirred for 1 hour, the insoluble matter was removed by filtration and the filtration was evaporated to dryness under reduced pressure. The residue was allowed to recrystallize from ethyl acetate to obtain 4-phenylbutyric acid N-hydroxysuccinimide ester (35 g) having a melting point of 82°C.

The above ester (13 g) was dissolved in chloroform (200 ml). This solution was added dropwise to a solution obtained by dissolving L-phenylalanine (16.5 g) and $Na_2CO_3$ (15.9 g) in water (150 ml) while stirring at room temperature. After that, the mixture was stirred for 7 hours and the insoluble matter was acidified to a pH of 1.0 with 6N HCl. The precipitated crystals were filtered, washed with water, and re-crystallized from 90% aqueous methanol to obtain N-(4-phenylbutyroyl)-L-phenylalanine (11.2 g); having a melting point of 178°C.

Elemental analysis:
Calc. C 73.28%, H 6.79%, N 4.49%
Found C 73.24%, H 6.94%, N 4.46%

Optical rotation:
$[\alpha]_D^{26}$= 8.33° (C = 1, acetone)

In the same manner, the compounds listed in the following Table 1 were obtained.

Table 1

| | Molecular Formula | m.p. (°C) | Optical rotation |
|---|---|---|---|
| S-24 | $C_{18}H_{16}ClNO_3$ | 155 - 158 | $[\alpha]_D^{30}$ - 31.02° (C = 1, MeOH) |
| S- 25 | " | 157 - 159 | $[\alpha]_D^{30}$ + 31.20° (C = 1, MeOH) |
| S- 27 | $C_{19}H_{19}NO_3$ | 135 - 140 | $[\alpha]_D^{25}$ - 35.89° (C = 1, MeOH) |
| S- 30 | $C_{19}H_{16}F_3NO_3$ | 158 - 160 | $[\alpha]_D^{20}$ - 19.10° (C = 1, MeOH) |
| S-31 | $C_{18}H_{16}FNO_3$ | 145 - 148 | $[\alpha]_D^{20}$ - 50.98° (C = 1, MeOH) |
| S-36 | $C_{19}H_{16}F_3NO_3$ | 157 - 160 | $[\alpha]_D^{20}$ + 19.03° (C = 1, MeOH) |

Example 3

Production of compound "S—11":

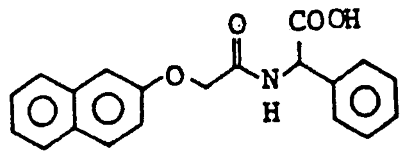

L-Phenylalaine (0.1 mole) was dissolved in 2N NaOH (50 ml) and ethyl ether (20 ml) was added thereto. To the mixture, while stirring vigorously with ice-cooling, the desired naphthoxyacetyl chloride (0.1 mole) and 2N NaOH (100 ml) in small portions were added. The mixture was stirred for 3 hours at a room temperature and washed once with ethyl ether. The aqueous layer was adjusted to a pH of 2 with 4N HCl to precipitate crude crystals. The crystals were placed on filter paper, dried and re-recrystallized with from ethylacetate-petroleum ether.

Compound "S—12" was produced in the same manner.

The results in respect of compounds "S—11" and "S—12" are given in the following Table 2.

Table 2

| Compound | Melting Point (°C) | $[\alpha]_D^{20}$ (C = 1, methanol) | Yield of Purified Crystals (%) |
|---|---|---|---|
| S-11 | 137 - 142 | -10.6° | 33 |
| S-12 | 173 - 176 | +25.1° | 36 |

Example 4

Production of compound "S—21":

Z-Phe$^{L}$-Phe$^{D}$

D-Phenylalanine (17/3 g) and $NaHCO_3$ (17.6 g) were added to water (150 ml).

N-Benzyloxycarbonyl-L-phenylalanine N-hydroxysuccinimide ester (27.7 g) was dissolved in tetrahydrofuran (150 ml), and the resulting solution was added to the aove aqueous solution at room temperature. The mxiture was reacted overnight. To the reaction solution, water (200 ml) was added and then the aqueous phase was adjusted to a pH of 2 with cooled 4N HCl. The deisred product was extracted with ethyl acetate (500 ml). The organic layer was washed first with 1N HCl and then with saturated aqueous NaCl solution, and dried over anhydrous magnesium sulphate. The solution was evaporated to dryness under reduced pressure. The resulting residue (28.0 g) was re-crystallized from ethyl acetate/n-hetane to obtain the required compound (20.3 g, yield: 65%).

The melting point and optical rotation of this compound is given in Table 3.

Table 3

| Compound | Molecular formula | m.p. (°C) | Optical Rotation |
|---|---|---|---|
| S-21 | $C_{26}H_{26}N_2O_5$ | 119 - 125 | $[\alpha]_D^{17}$= -34.2° (C =1,EtOH) |

Example 5

The absorption promoters listed in Table 4 were dissolved or suspended in 0.5% CMC—0.05M tris-HCl buffers (pH:7.8) and the solutions or suspensions were mixed with aqueous insulin solution. Female ICR—CD—1 mice, 5 to 7 weeks old, were orally administered with predetermined amounts of the mixtures. A predetermined duration later, the percent decrease in blood glucose and the degree of elevation of blood insulin as compared with a control group were measured. The results are shown in Table 4.

The symbol "Z" in the structural formulae in the Table (and in Example 4 above) represents a benzyloxycarbonyl group. The upper figures in the right hand columns represent the percent decrease in blood glucose and the lower parenthesized figures indicate the degree of elevation in blood insulin.

Table 4

| Compound | Structure | Dose with Insulin 2.5 U/10 g (Body Weight) | 1) Decrease in Blood Glucose (%) 2) Degree of Elevation of Blood Insulin — Time (minutes) 30 | 60 |
|---|---|---|---|---|
| S-1 | 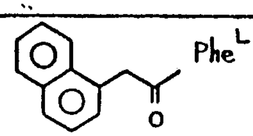 | 6.0 mg/10 g | 84.9 (56.4) | 75.4 (60.0) |
| S-2 | 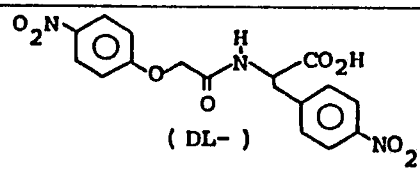 ( DL- ) | 6.0 mg/10 g | 23.2 (>119) | 60.0 (89.7) |
| S-3 | 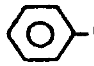-CH=CH-CO-$Phe^L$ | 6.0 mg/10 g | 84.4 (34.2) | 84.3 (68.1) |
| S-4 | 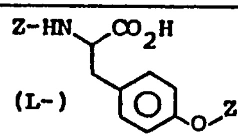 (L-) | 3.0 mg/10 g | 52.7 (6.54) | 31.1 (2.04) |
| S-5 | 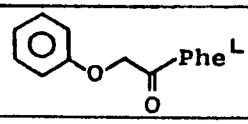 | 12.5 mg/10 g | 57.9 (88.5) | 33.5 (18.0) |
| S-6 | 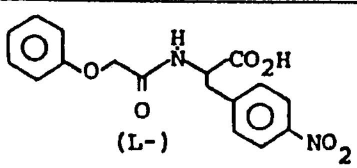 (L-) | 3.1 mg/10 g | 21.5 (20.6) | 44.7 (21.8) |
| S-7 | Z - $Phe^L$ - $Phe^L$ | 3.0 mg/10 g | 70.0 (7.72) | 40.6 (9.35) |

Table 4 (Continued)

| Compound | Structure | Dose with Insulin 2.5 U/10 g (Body Weight) | 1) Decrease in Blood Glucose (%) 2) Degree of Elevation of Blood Insulin | |
|---|---|---|---|---|
| | | | Time (minutes) | |
| | | | 30 | 60 |
| S-8 | 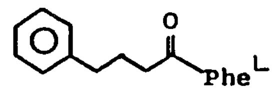 | 6.0 mg/10 g | 57.1 (7.31) | 35.6 (9.64) |
| S-9 | 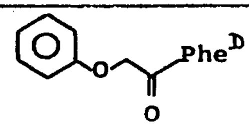 | 3.0 mg/10 g | 37.3 (11.8) | 29.7 (2.67) |
| S-10 | Z-$Phe^{L}$-$Tyr^{L}$ | 6.0 mg/10 g | 61.5 (41.9) | 43.4 (4.85) |
| S-11 | 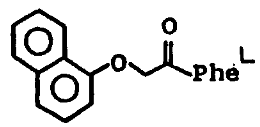 | 6.0 mg/10 g | 54.0 (11.8) | 59.7 (5.07) |
| S-12 | 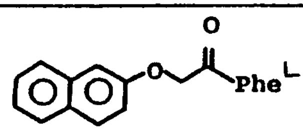 | 6.0 mg/10 g | 50.8 (6.05) | 66.8 (5.62) |
| S-13 | 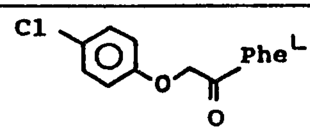 | 1.5 mg/10 g | 31.9 (43.4) | 18.5 (9.82) |
| S-14 | 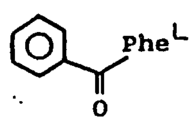 | 6.0 mg/10 g | 35.2 (6.31) | 37.8 (3.31) |

Table 4 (Continued)

| Compound | Structure | Dose with Insulin 2.5 U/10 g (Body Weight) | 1) Decrease in Blood Glucose (%) 2) Degree of Elevation of Blood Insulin | |
|---|---|---|---|---|
| | | | Time (minutes) | |
| | | | 30 | 60 |
| S-15 | Z - $Phe^L$ | 3.0 mg/10 g | 37.5 (3.43) | 60.7 (2.36) |
| S-16 | Z - $Tyr^L$ | 6.0 mg/10 g | 31.0 (50.0) | 27.2 (2.99) |
| S-17 | 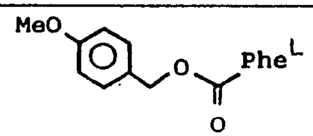 | 3.0 mg/10 g | 64.8 (14.4) | 50.6 (21.2) |
| S-18 | Z - $Phe^D$ | 6.0 mg/10 g | 52.3 (6.79) | 67.2 (10.6) |
| S-20 | Z - $Phe^D$ - $Phe^L$ | 1.5 mg/10 g | 22.4 (2.33) | 32.7 (2.46) |
| S-21 | Z - $Phe^L$ - $Phe^D$ | 6.0 mg/10 g | 42.6 (14.9) | 32.7 (5.26) |

Table 4 (Continued)

| Compound | Structure | Dose with Insulin 2.5 U/10 g (Body Weight) | 1) Decrease in Blood Glucose (%) 2) Degree of Elevation of Blood Insulin — Time (minutes) 30 | 60 |
|---|---|---|---|---|
| S-24 | 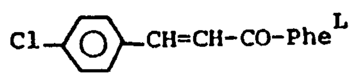 | 1.5 mg/10 g | 37.1 (5.46) | 27.4 (3.04) |
| S-25 | 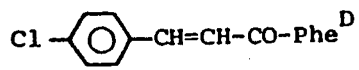 | 1.5 mg/10 g | 34.9 ( - ) | 29.5 ( - ) |
| S-27 | 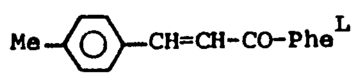 | 1.5 mg/10 g | 61.5 (7.76) | 34.4 ( 1.0) |
| S-30 | 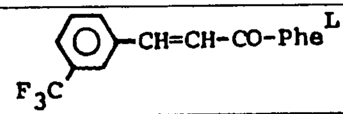 | 3.0 mg/10 g | 48.8 ( - ) | 44.4 ( - ) |
| S-31 | 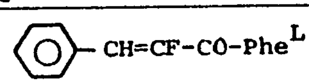 | 3.0 mg/10 g | 33.5 ( - ) | 72.5 ( - ) |
| S-36 | 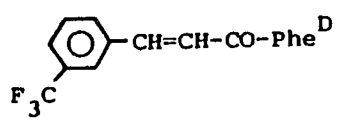 | 3.0 mg/10 g | 52.0 ( - ) | 49.1 ( - ) |

Table 4 shows the effectiveness of the absorption promoters of the present invention administered orally, but it should be understood that the same results are obtained by using the absorption promoters as a conventional suppository preparation, together with insulin. The absorption promoters of the present invention are very useful in that they enable clinical insulin therapy by the oral or perenteral (e.g. rectal) administration.

Example 6

Toxicity studies of phenylalanine derivatives of the invention, in respect of their oral IN potentiating activity in female CD—1(ICR) mice, were carried out, the derivatives being suspended in 0.5% CMC. The results are given in the following Table 5.

Table 5

| Compound | $LD_{50}$ (mg/kg) |
|---|---|
| N-Phenoxyacetyl-L-phenylalanine | >4000 |
| N-(1-Naphthyloxy)acetyl-L-phenylalanine | >4000 |
| N-(2-Naphthyloxy)acetyl-L-phenylalanine | >4000 |
| N-(4-Chlorophenoxy)acetyl-L-phenylalanine | >3500 |
| N-Benzyloxycarbonyl-L-phenylalanine | >2750 |
| N-Benzyloxycarbonyl-D-phenylalanine | >4000 |
| N-p-Methoxybenzyloxycarbonyl-L-phenylalanine | 750 |
| N-Benzyloxycarbonyl-L-phenylalanyl-L-phenylalanine | >3000 |
| N-Benzyloxycarbonyl-L-phenylalanyl-L-tyrosine | >3000 |
| N-Cinnamoyl-L-phenylalanine | >2500 |

Example 7 (Preparation of Tablets)

Porcine insulin (0.577 g, 15000 U, Zn content = 0.5%) was dissolved in 0.05 N HCl (30 ml) and the thus obtained solution was diluted with distilled water (30 ml).

The compound "S—27" (6 g) was dissolved in 0.1N NaOH (200 ml) and the pH was adjusted to 7.5 by the addition of 0.1N HCl. The solution was diluted with phosphate buffer (0.02M, pH 7.5) to a volume of 600 ml.

The insulin solution produced as above was added dropwise to the "S—27" solution maintained at 20°C an with vigorously stirring, the resulting solution was adjusted to pH 7.5, and immediately was freeze-dried.

Tablets were prepared from the freeze-dried material (25 mg), pregelatinized starch (82 mg), microcrystalline cellulose (82 mg), and magnesium stearate (1 mg). Then, enteric coating tablets were prepared by a conventional method using an aqueous solution of hydroxyphenylmethylcellulose (8%) as a pre-coating agent for forming an undercoat and an aqueous solution of hydroxypropylmethylcellulose phthalate (10%) and polyacetyne (3%) as a coating agent.

Example 8 (Preparation of capsules)

To glacial acetic (250 ml), a compound of the invention (30 g) was added and dissolved by heating. Porcine insulin (2 g, 52000U, Zn content = 0.5%) was added in small portions to the above solution cooled to 20°C, with stirring, and dissolved therein. From the solution the acetic acid was distilled off under reduced pressure and at the same temperature.

To thus obtained solid residue, n-hexane (100 ml) was added, and the solid residue was pulverized, put on a filter, and washed with n-hexane. Then the n-hexane adhering to the powder was evaporated under reduced pressure. The powder was dried under reduced pressure in the presence of solid NaOH.

Dry packed capsules containing 50 mg per capsule of active ingredient were prepared:

| | |
|---|---|
| the above powder | 50 mg |
| lactose | 149 mg |
| magnesium stearate | 1 mg |
| capsule | 200 mg |

The powder was first reduced to a No. 60 powder, and the lactose and magnesium stearate were passed through a No. 60 sieve cloth to fall onto the powder and mixed sufficiently with it. The mixture was packed into No. 1 dry gelatin capsules.

**Claims**

1. A phenylalanine derivative having the following general formula:

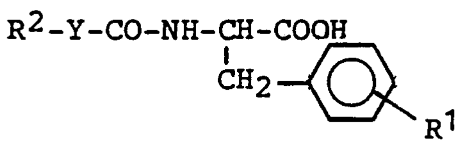

wherein $R^1$ is a hydrogen atom, a nitro group, a hydroxyl group or a hydroxyl group protected by an esterifying group, —Y— is a straight bond, $C_1$ to $C_{10}$ alkylene group, a vinylene group, or a group having the formula $—CH_2—O—$ or $—O—CH_2—$, and $R^2$ is a phenyl or naphthyl group; or the group $R^2—Y—CO—$ is an N-benzyloxycarbonylphenylalanyl group; or a non-toxic salt thereof.

2. N-(4-chlorocinnamoyl)-L-phenylalanine.

3. N-(4-chlorocinnamoyl)-D-phenylalanine.

4. N-(4-methylcinnamoyl)-L-phenylalanine.

5. N-(3-trifluoromethylcinnamoyl)-L-phenylalanine.

6. N-(α-fluorocinnamoyl)-L-phenylalanine.

7. N-(4-trifluoromethylcinnamoyl)-D-phenylalanine.

8. The use of a phenylalanine derivative as claimed in any of claims 1 to 7, or a non-toxic salt thereof, in the preparation of a promoting agent for promoting the absorption of insulin.

9. A pharmaceutical composition comprising (a) insulin and (b) a phenylalanine derivative as claimed in any of claims 1 to 7, or a non-toxic salt thereof.

**Patentansprüche**

1. Phenylalaninderivat mit der folgenden allgemeinen Formel

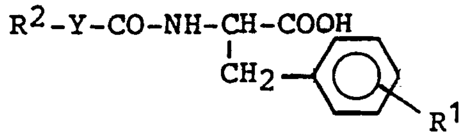

in der $R^1$ ein Wasserstoffatom, eine Nitrogruppe, eine Hydroxylgruppe oder eine Hydroxylgruppe, die mit einer veresternden Gruppe geschützt ist, bedeutet, —Y— eine einfache Bindung, eine $C_1$ bis $C_{10}$-Alkylengruppe, eine Vinylengruppe, oder eine Gruppe der Formel $—CH_2—O—$ oder $—O—CH_2—$ bedeutet und $R^2$ eine Phenyl- oder Naphthylgruppe bedeutet; oder die Gruppe $R^2—Y—CO—$ eine N-Benzyloxycarbonylphenylalanylgruppe bedeutet; oder ein nichttoxisches Salz davon.

2. N-(4-Chlorcinnamoyl)-L-phenylalanin.

3. N-(4-Chlorcinnamoyl)-D-phenylalanin.

4. N-(4-Methylcinnamoyl)-L-phenylalanin.

5. N-(3-Trifluoromethylcinnamoyl)-L-phenylalanin.

6. N-(α-Fluorcinnamoyl)-L-phenylalanin.

7. N-(4-Trifluormethylcinnamoyl)-D-phenylalanin.

8. Verwendung eines Phenylalaninderivats nach einem der Ansprüche 1 bis 8 oder eines nichttoxischen Salzes davon bei der Herstellung eines Förderungsmittels zur Förderung der Absorption von Insulin.

9. Pharmazeutische Zusammensetzung, enthaltend (a) Insulin und (b) Phenylalaninderivat nach einem der Ansprüche 1 bis 7 oder ein nicht-toxisches Salz davon.

**Revendications**

1. Un dérivé de phénylalanine répondant à la formule générale suivant:

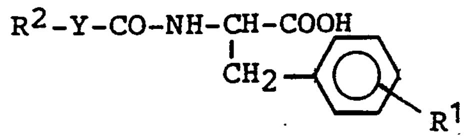

dans laquelle $R^1$ est un atome d'hydrogène, un groupe nitro, un groupe hydroxyle ou un groupe hydroxyle protégé par un groupe estérifiant, —Y— est une liaison directe un groupe alkylène en $C_1—C_{10}$, un groupe vinylène ou un groupe de formule $—CH_2—O—$ ou $—O—CH_2—$ et $R^2$ est un groupe phényle ou naphtyle; ou le groupe $R^2—Y—CO—$ est un groupe N-benzoxycarbonylphénylalanyle; ou leurs sels non toxiques.

2. La N-(4-chlorocinnamoyl)-L-phénylalanine.

3. La N-(4-chlorocinnamoyl)-D-phénylalanine.

4. La N-(4-méthylcinnamoyl)-L-phénylalanine.

5. La N-(3-trifluorométhylcinnamoyl)-L-phénylalanine.

6. La N-(α-fluorocinnamoyl)-L-phénylalanine.

7. La N-(3-trifluorométhylcinnamoyl)-D-phénylalanine.

8. L'utilisation d'un dérivé de phénylalanine selon l'une quelconque des revendications 1 à 7, ou d'un de leurs sels non toxiques, dans la préparation d'un agent promoteur pour favoriser l'absorption de l'insuline.

9. Une composition pharmaceutique comprenant (a) de l'insuline et (b) un dérivé de phénylalanine selon l'une quelconque des revendications 1 à 7, ou un de ses sels non toxiques.